# EUROPEAN PATENT APPLICATION

(11) **EP 4 659 759 A1**
(43) Date of publication of application: **10.12.2025**
(21) Application number: 24179919.6
(22) Date of filing: 04.06.2024
(51) Int. Cl.: A61K 38/18, A61K 38/17, A61P 43/00

(54) **ADMINISTRATION OF NGF FOR THE TREATMENT OF NIEMANN-PICK TYPE C DISEASE**

(71) Applicant: Dompé farmaceutici SpA, 20122 Milano (IT)
(72) Inventor: SIRICO, Anna, 67100 L'Aquila (IT); DE LUCIA, Maria, 80131 Napoli (IT); CYPRIANO DUTRA, Rafael, 80131 Napoli (IT); SEGATTO, Marco, 86100 Campobasso (IT)
(74) Representative: Dompé farmaceutici Spa

(57) **Abstract**

The present invention relates to Nerve growth factor (NGF) or a mutein thereof, for use in the treatment of Niemann-Pick type C in a subject.

## Description

### FIELD OF THE INVENTION

The present invention relates to the administration of NGF for the treatment of Niemann-Pick type C disease in a subject.

### STATE OF THE ART

Niemann Pick type C (NPC) is a rare autosomal recessive neurovisceral lipid storage disease, characterized by an aberrant accumulation of free (i.e. un-esterified) lipids, mainly cholesterol, within the lysosomes of the affected individuals.

The essential role of cholesterol in maintaining functional integrity of virtually all types of cells is well-known in the art.

In wild-type human cells, most of the cholesterol exists in ester form; in fact, cholesterol esterification is one of the several mechanisms cells use to prevent an intracellular accumulation of free cholesterol, that is toxic to cells.

Moreover, cholesterol in ester form can be stored and transported more efficiently, because cholesteryl esters are more hydrophobic than cholesterol, and consequently they can be packaged inside the hydrophobic core of lipoproteins, including low-density lipoproteins (LDL), and transported throughout the body.

In wild-type cells, LDL particles, which are mostly composed of cholesterol esters, are intracellularly delivered to the lysosome, where the cholesterol esters are cleaved by lysosomal acid lipase to release free cholesterol for cellular use.

Niemann Pick type C (NPC) is caused by mutations in the NPC type C1 (NPC1) and NPC type C2 (NPC2) genes; in particular, mutations in NPC1 are responsible for the vast majority (95%) of NPC clinical cases.

NPC1 and NPC2 proteins are glycoproteins localized in the lysosomes, and involved in the intracellular trafficking of cholesterol. In particular, these proteins are essential for the export of the free cholesterol from lysosomes to the cell cytoplasm.

Mutations in NPC1 and/or NPC2 gene(s) associated with NPC result in expression of NPC1 and/or NPC2 proteins with impaired or complete loss of their function.

In particular, cells harboring inactivating mutations in NPC1 are defective in the delivery of newly hydrolyzed low-density lipoprotein (LDL) cholesterol from the lysosomes, demonstrate impaired rates of esterification of LDL cholesterol, and aberrantly accumulate unesterified cholesterol in late endosomal/lysosomal compartment (Millard E. et al., The Sterol-sensing Domain of the Niemann-Pick C1 (NPC1) Protein Regulates Trafficking of Low Density Lipoprotein Cholesterol, Journal of Biological Chemistry, Volume 280, Issue 31, 2005, Pages 28581-28590).

Moreover, depending on the NPC1 gene mutations, NPC1 mutated protein is only partially trafficked correctly to the lysosomes, but it is predominantly localized to the endoplasmic reticulum (ER), or even entirely retained in the ER (Shammas, H. et al., Different Niemann-Pick C1 Genotypes Generate Protein Phenotypes that Vary in their Intracellular Processing, Trafficking and Localization. Sci Rep 9, 5292 (2019)).

Furthermore, these proteins are often recognized as "non-self" by the cell, and therefore they are generally degraded intracellularly.

Thus, according to the above, NPC cells are subjected to a perturbation of the proper functioning of intracellular cholesterol-sensing mechanisms, which leads to an overall dysbalance in the regulatory network of cholesterol homeostasis, thus resulting in an abnormal accumulation of free cholesterol into the lysosomes, including neuronal lysosomes (Wheeler S, Sillence DJ. Niemann-Pick type C disease: cellular pathology and pharmacotherapy. J Neurochem. 2020 Jun;153(6):674-692).

Among the cell cholesterol-sensing mechanisms perturbed in NPC cells, SREBP2 (sterol regulatory element binding protein 2) pathway is particularly relevant.

In fact, the lack of sufficient free cholesterol in NPC cell cytoplasm induces the activation and/or up-regulation of cell pathways of lipid synthesis and uptake, in order to match the need of lipids of the cell.

For example, SREBP2 undergoes a proteolytic cleavage leading to a nuclear transcriptionally active fragment, that migrates into the nucleus to activate the transcription of target genes involved in cholesterol synthesis and uptake, including hydroxymethylglutaryl coenzyme A reductase (HMGCR) and LDLR (Madison BB. Srebp2: A master regulator of sterol and fatty acid synthesis. J Lipid Res. 2016 Mar;57(3):333-5; Malara M. et al., Endo-lysosomal dysfunction and neuronal-glial crosstalk in Niemann-Pick type C disease. Philos Trans R Soc Lond B Biol Sci., 2024 Apr 8;379(1899):20220388), thus intensifying the abnormal intracellular free sterol accumu lation.

One of the most crucial consequence of cholesterol accumulation in NPC patient is the effect on their neurons, which become distorted. In fact, NPC patients often have ectopic dendrite formation and neurofibrillary tangles similar to those see in AD. In particular, the Purkinje cells of the cerebellum seem particularly vulnerable to the effects of cholesterol accumulation, perhaps related to the capacity of the cerebellum to synthesize cholesterol (Björkhem I. et al., Genetic connections between neurological disorders and cholesterol metabolism, 2010, J. Lipid Res.51: 2489-2503).

Besides cholesterol, glycosphingolipids, sphingomyelin and sphingosine accumulate in NPC disease, suggesting a broader defect in the homeostasis of diverse lipid species (Malara M. et al., Endo-lysosomal dysfunction and neuronal-glial crosstalk in Niemann-Pick type C disease. Philos Trans R Soc Lond B Biol Sci., 2024 Apr 8;379(1899):20220388.)

The complexity of NPC disease has led to many different therapeutic approaches, in order to reduce the sterol accumulation in cells, in particular in neurons.

The main current therapies include the administration of Miglustat (OGT 918, N-butyl-deoxynojirimycin, which is an iminosugar acting as an inhibitor of glucosylceramide synthase, needed in the early stages of glycosphingolipid synthesis), HP-β-CD (2-Hydroxypropyl-β-cyclodextrin, a cyclic oligosaccharide initially absorbed into the endolysosome where it transports unesterified cholesterol to the cytosol and reduces its accumulation in the endolysosomes independently of NPC1 and NPC2 proteins), Vorinostat (an inhibitor of histone deacetylases that reduces the accumulation of lipids in the lysosomes of cultured skin fibroblasts of NPC patients), or gene therapy (Sitarska D. et al., Treatment trials in Niemann-Pick type C disease. Metab Brain Dis. 2021 Dec;36(8):2215-2221).

Unfortunately, the above available treatments are characterized by several adverse effects and risks, which can outweigh the potential benefits they may provide.

In fact, Miglustat often causes gastrointestinal disturbances, including diarrhea, flatulence, bloating, abdominal discomfort, nausea and vomiting (Belmatoug N. et al., Gastrointestinal disturbances and their management in miglustat-treated patients. J Inherit Metab Dis. 2011 Oct;34(5):991-1001); HP-β-CD is not able to cross the blood-brain barrier (BBB) when administered systemically, possibly because of its large size; and Vorinostat barely penetrates the BBB, the concentration of Vorinostat in the brain being in fact 100-fold lower than in the plasma (Sitarska D. et al., Treatment trials in Niemann-Pick type C disease. Metab Brain Dis. 2021 Dec;36(8):2215-2221).

It is therefore strongly felt the need of developing new effective, long lasting and safe therapeutic approaches for the treatment of Niemann Pick type C disease, overcoming the adverse effects and disadvantages of the above main current therapy approaches.

### SUMMARY OF THE INVENTION

The present inventors have now surprisingly found that nerve growth factor (NGF), as well as brain-derived nerve factor (BDNF), significantly reduce cholesterol buildup in NPC cells, thus counteracting the aberrant cholesterol intracellular accumulation within NPC cells.

Moreover, the present inventors have also found that NGF is particularly effective in improving the protein expression and the correct localization of NPC1 in the cell lysosomes, as well as in restoring the alterations in the expression of some of the main cholesterol regulatory proteins, in particular SREBP2 protein expression.

Furthermore, the inventors have found that when NGF or BDNF is administered intranasally, they are unexpectedly effective in decreasing the abnormal accumulation of sterols, in particular free cholesterol, into the NPC cells in the CNS.

Thus, NGF and BDNF were found to be particularly effective in treating the NPC disease, in particular when administered intranasally to the NPC subject.

Accordingly, a first object of the invention is NGF or a mutein thereof, for use in the treatment of Niemann Pick Type C (NPC) disease in a subject.

Another object of the the invention is BDNF for use in the treatment of Niemann Pick Type C (NPC) disease in a subject.

A further object of the invention is a pharmaceutical composition comprising NGF or a mutein thereof, or BDNF, for use in the treatment of Niemann Pick Type C (NPC) disease in a subject.

### BRIEF DESCRIPTION OF THE FIGURES

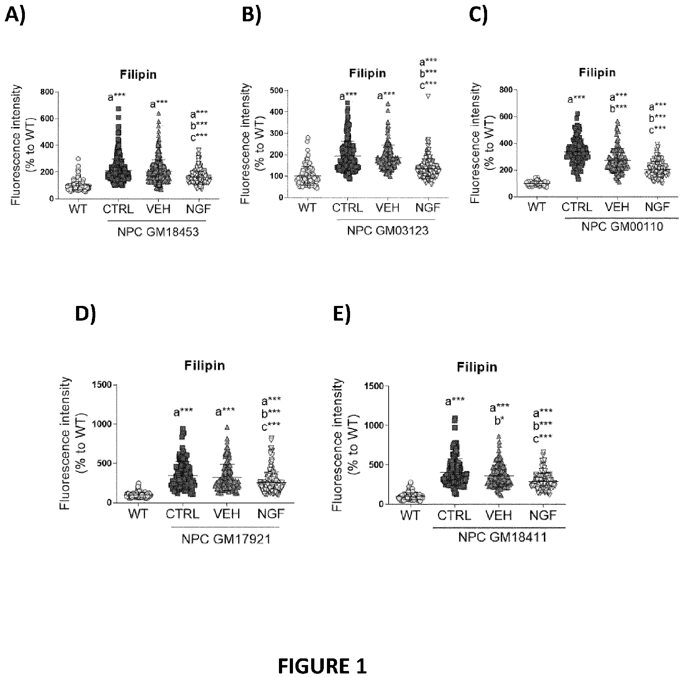

**Figure 1** shows fluorescence intensity graphs of filipin staining analyzed from each cell (dots) of:
   - WT (GM05659), NPC GM18453 control (CTRL), NPC GM18453 vehicle-treated (VEH) and NPC GM18453 rhNGF-treated (NGF) cell lines (panel a));
   - WT (GM00726), NPC GM03123 control (CTRL), NPC GM03123 vehicle-treated (VEH) and NPC GM03123 rhNGF-treated (NGF) cell lines (panel b));
   - WT (GM05659), NPC GM00110 control (CTRL), NPC GM00110 vehicle-treated (VEH) and NPC GM00110 rhNGF-treated (NGF) cell lines (panel c));
   - WT (GM05659), NPC GM17921 control (CTRL), NPC GM17921 vehicle-treated (VEH) and NPC GM17921 rhNGF-treated (NGF) cell lines (panel d)); and
   - WT (GM00726), NPC GM18411 control (CTRL), NPC GM18411 vehicle-treated (VEH) and NPC GM18411 rhNGF-treated (NGF) cell lines (panel e)), according to Example 1, said dots being derived from 15 images taken over at least 3 independent experiments. Data represent means ± SD. Statistical analysis was assessed by using one-way ANOVA, followed by Tukey's post hoc test. * p < 0.05, ** p < 0.01, *** p < 0.001. "a" indicates statistical significance vs WT group; "b" indicates statistical significance vs CTRL group; "c" indicates statistical significance vs VEH group.

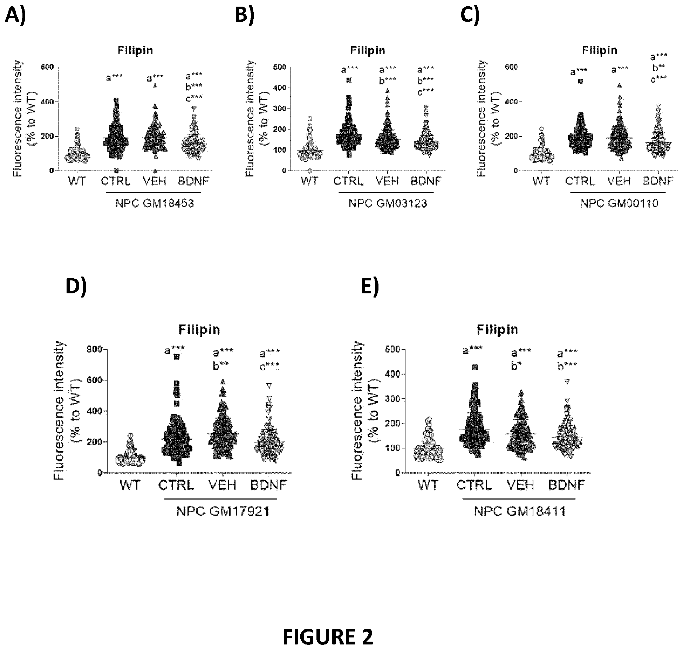

**Figure 2** shows fluorescence intensity graphs of filipin staining analyzed from each cell (dots) of:
   - WT (GM05659), NPC GM18453 control (CTRL), NPC GM18453 vehicle-treated (VEH) and NPC GM18453 rhBDNF-treated (BDNF) cell lines (panel a));
   - WT (GM00726), NPC GM03123 control (CTRL), NPC GM03123 vehicle-treated (VEH) and NPC GM03123 rhBDNF-treated (BDNF) cell lines (panel b));
   - WT (GM05659), NPC GM00110 control (CTRL), NPC GM00110 vehicle-treated (VEH) and NPC GM00110 rhBDNF-treated (BDNF) cell lines (panel c));
   - WT (GM05659), NPC GM17921 control (CTRL), NPC GM17921 vehicle-treated (VEH) and NPC GM17921 rhBDNF-treated (BDNF) cell lines (panel d)); and
   - WT (GM00726), NPC GM18411 control (CTRL), NPC GM18411 vehicle-treated (VEH) and NPC GM18411 rhBDNF-treated (BDNF) cell lines (panel e)), according to Example 1, said dots being derived from 15 images taken over at least 3 independent experiments. Data represent means ± SD. Statistical analysis was assessed by using one-way ANOVA, followed by Tukey's post hoc test. * p < 0.05, ** p < 0.01, *** p < 0.001. "a" indicates statistical significance vs WT group; "b" indicates statistical significance vs CTRL group; "c" indicates statistical significance vs VEH group.

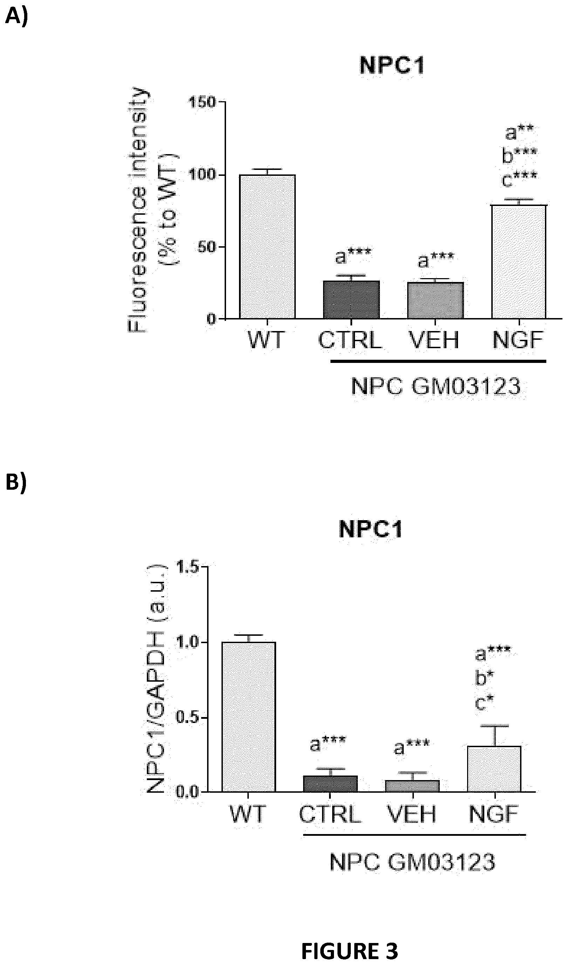

**Figure** 3 shows bar graphs of the results of the immunofluorescence (expressed by fluorescence intensity % to WT) (panel a)), and Western blot analysis (intensity over the housekeeping GAPDH, (panel b)) performed on GM00726 (WT), NPC GM03123 control (CTRL), NPC GM03123 vehicle-treated (VEH), and NPC GM03123 rhNGF-treated (NGF) cell lines, according to Example 1. N=6 for immunofluorescence analysis; N=3 for Western blot analysis. Data represent means ± SD. Statistical analysis was assessed by using one-way ANOVA, followed by Tukey's post hoc test. *p<0.05, **p<0.01, ***p<0.001. "a" indicates statistical significance vs WT group; "b" indicates statistical significance vs CTRL group; "c" indicates statistical significance vs VEH group.

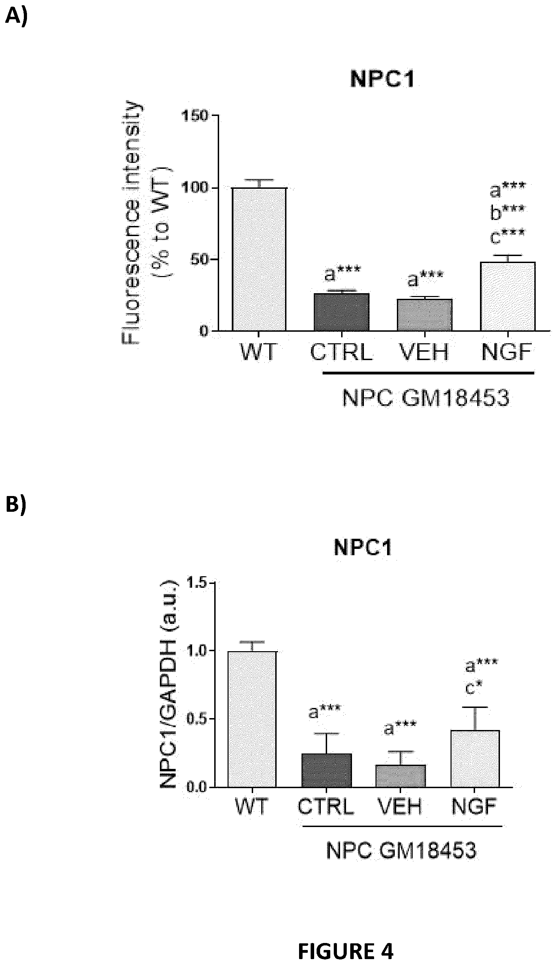

**Figure 4** shows bar graphs of the results of the immunofluorescence (expressed by fluorescence intensity % to WT) (panel a)) and Western blot (intensity over the housekeeping GAPDH, panel b)) analysis performed on GM05659 (WT), NPC GM18453 control (CTRL), NPC GM18453 vehicle-treated (VEH), and NPC GM18453 rhNGF-treated (NGF) cell lines, according to Example 1. N=6 for immunofluorescence analysis; N=3 for Western blot analysis. Data represent means ± SD. Statistical analysis was assessed by using one-way ANOVA, followed by Tukey's post hoc test. *p<0.05, **p<0.01, ***p<0.001. "a" indicates statistical significance vs WT group; "b" indicates statistical significance vs CTRL group; "c" indicates statistical significance vs VEH group.

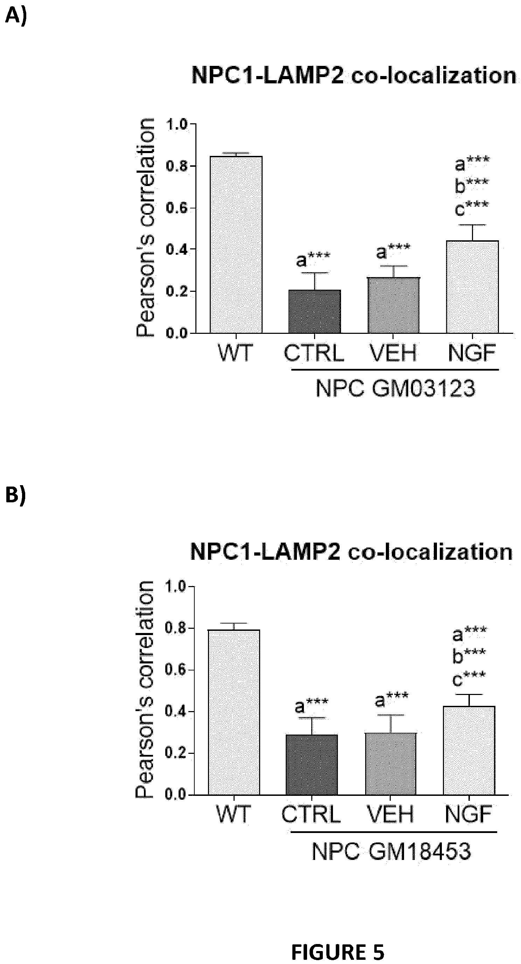

**Figure 5** shows bar graphs of the results of the Pearson's correlation analysis of NPC1 and LAMP2, performed on GM00726 (WT), NPC GM03123 control (CTRL), NPC GM03123 vehicle-treated (VEH), and NPC GM03123 rhNGF-treated (NGF) cell lines (panel a)), and on GM05659 (WT), NPC GM18453 control (CTRL), NPC GM18453 vehicle-treated (VEH), and NPC GM18453 rhNGF-treated (NGF) cell lines (panel b)), according to Example 1. N=6 independent experiments. Data represent means ± SD. Statistical analysis was assessed by using one-way ANOVA, followed by Tukey's post hoc test. * p < 0.05, ** p < 0.01, *** p < 0.001. "a" indicates statistical significance vs WT group; "b" indicates statistical significance vs CTRL group; "c" indicates statistical significance vs VEH group.

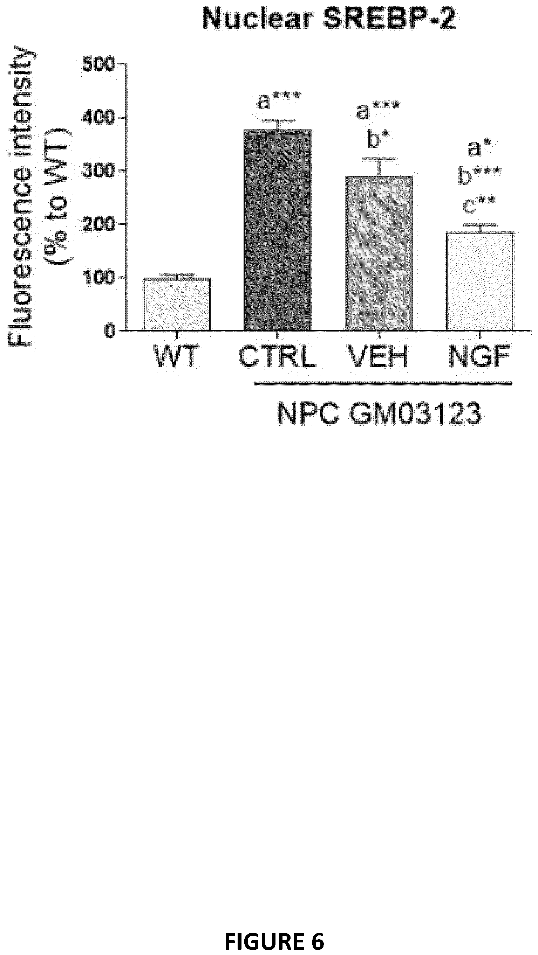

**Figure 6** shows bar graphs of the results of the immunofluorescence analysis (expressed by fluorescence intensity % to WT) of nuclear SREBP2 performed on GM00726 (WT), NPC GM03123 control (CTRL), NPC GM03123 vehicle-treated (VEH), and NPC GM03123 rhNGF-treated (NGF) cell lines. N=6 independent experiments. Data represent means ± SD. Statistical analysis was assessed by using one-way ANOVA, followed by Tukey's post hoc test. * p < 0.05, ** p < 0.01, *** p < 0.001. "a" indicates statistical significance vs WT group; "b" indicates statistical significance vs CTRL group; "c" indicates statistical significance vs VEH group.

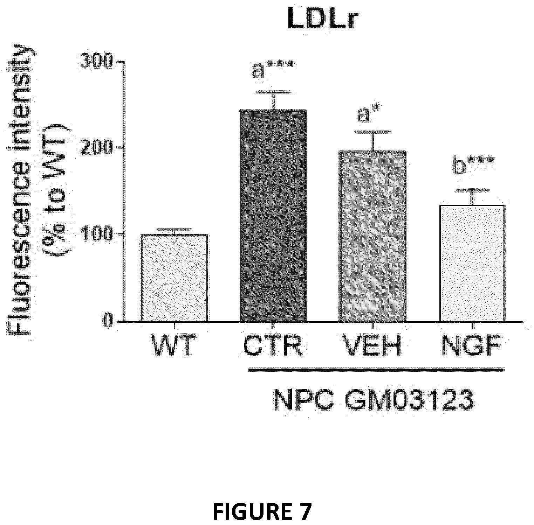

**Figure 7** shows bar graphs of the results of the immunofluorescence analysis (expressed by fluorescence intensity (% to WT)) of LDLR performed on GM00726 (WT), NPC GM03123 control (CTRL), NPC GM03123 vehicle-treated (VEH), and NPC GM03123 rhNGF-treated (NGF) cell lines. N=6 independent experiments. Data represent means ± SD. Statistical analysis was assessed by using one-way ANOVA, followed by Tukey's post hoc test. * p < 0.05, ** p < 0.01, *** p < 0.001. "a" indicates statistical significance vs WT group; "b" indicates statistical significance vs CTRL group; "c" indicates statistical significance vs VEH group.

### DETAILED DESCRIPTION OF THE INVENTION

According to a preferred embodiment, the present invention relates to a nerve growth factor (NGF) or a mutein thereof, for use in the treatment of Niemann Pick Type C (NPC) disease in a subject.

Preferably, the NGF or a mutein thereof is administered intranasally to the subject.

The term "treatment", as used herein, refers to the eradication/amelioration of a disorder, or of one or more of the symptoms associated thereof.

Preferably, said subject is a human subject.

Preferably, said subject has been diagnosed with NPC disease, and said NGF or mutein thereof is for use in the treatment of said disease, preferably by intranasal administration to said subject.

Preferably, said NGF is human NGF.

Preferably, said human NGF has the aminoacid sequence of SEQ. ID NO.1 below SEQ. ID NO.1:

SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVR

Alternatively, said human NGF has the aminoacid sequence of SEQ ID NO. 2 below: SEQ ID NO. 2:

SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVRRA

Alternatively, said human NGF is a mixture of NGFs having aminoacid sequences of SEQ ID NO. 1 and SEQ ID NO. 2.

The human NGF of SEQ ID NO. 2 have an aminoacid sequence that only differ from the NGF of SEQ ID NO. 1 for the presence of two additional amminoacids at the C-terminus. Both forms of NGF are found in human cells and therefore are considered as wild type human NGF.

Therefore, when referring to "human NGF" or "wild type human NGF" in the present application, it is meant a human NGF of SEQ ID NO: 1 or SEQ ID NO: 2.

Preferably, said NGF is produced by recombinant DNA technology, preferably it is a human recombinant NGF (rhNGF). Methods of producing rhNGF are known to the person skilled in the art, for example those described in WO0022119A1 and WO2013092776A1.

Preferably, said NGF has a purity higher than 70%, more preferably higher than 80%, higher than 90%, higher than 95%, higher than 98% or higher than 99%. The purity of NGF may be determined by conventional means known to those skilled in the art, for example by HPLC analysis.

The term "mutein of NGF" refers to a biologically active mutein of NGF, meaning a NGF protein having an aminoacid sequence with one or more aminoacid mutations, preferably substitutions, such that the therapeutic activity of wild type NGF is maintained.

Preferably, said mutein is a mutein of wild type human NGF.

Particularly preferred for use according to the invention, is a mutein of NGF, wherein the mutein is characterized by more than 70%, more preferably more than 80%, even more preferably more than 90%, and most preferably more than 95% sequence identity with wild type human NGF.

Preferably, the mutein is a mutein of wild type human NGF characterized by at least one mutation, preferably aminoacid substitution of proline at position 61 by another amino acid, in the sequence of wild type human NGF. In a particularly preferred embodiment, proline at position 61 is substituted by serine.

Preferably, the mutein is a mutein of human NGF, characterized by at least one mutation of the amino acid sequence associated with reduced nociceptive activity. More preferably said mutein is characterized by at least one mutation, preferably amino acid substitution, at any of positions 95-101 of wild type human NGF. Even more preferably, said mutein is characterized by substitution of the arginine in position 100 of wild type human NGF. Most preferably, arginine at position 100 of wild type human NGF is substituted by glutamic acid.

According to the invention, particularly preferred NGF muteins have the aminoacid sequences of SEQ ID NO. 3-6 below:
SEQ ID NO. 3: 
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWEFIRIDTACVCVLSRKA
VR
SEQ ID NO. 4: 
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWEFIRIDTACVCVLSRKA
VRRA
SEQ ID NO. 5: 
SSSHPIFHRGEFSVCDSVSVWVGDKTTATDIKGKEVMVLGEVNINNSVFKQYFFETKC
RDSNPVDSGCRGIDSKHWNSYCTTTHTFVKALTMDGKQAAWEFIRIDTACVCVLSRKA
VR
SEQ ID NO. 6:

SSSHPIFHRGEFSVCDSVSVWVGDKTTATDIKGKEVMVLGEVNINNSVFKQYFFETKC
RDSNPVDSGCRGIDSKHWNSYCTTTHTFVKALTMDGKQAAWEFIRIDTACVCVLSRKA
VRRA

As used herein, the expressions "NGF has the aminoacid sequence of" and "muteins have the aminoacid sequences of" mean that NGF and the muteins thereof consist of the above-disclosed aminoacid sequences, respectively.

The above described muteins are particularly advantageous for use according to the invention since they maintain the same bioactivity than wild type human NGF but are able to induce a lower nociceptive sensitivity compared to the corresponding wild type human NGF. Preferably, said mutein of human NGF is produced by recombinant DNA technology. Methods of producing muteins of rhNGF according to the invention by recombinant DNA technology are known to the person skilled in the art, for example those described in WO2019/207106.

Preferably, the NGF or mutein for use according to the invention is administered to the subject daily or every two/three days throughout the period of treatment.

More preferably, the NGF or mutein for use according to the invention is administered to the subject from one to three times a day.

Preferably, said period of treatment is a period of between 7 and 90 days, preferably between 15 and 60 days.

Preferably, the amount of NGF or mutein thereof per each administration is between 5 µg and 1 mg, more preferably between 10 µg and 400 µg, even more preferably between 15 µg and 200 µg, most preferably about 20 µg.

The effective amount of said NGF or mutein used in each administration, the duration of the treatment terapy, and the number of administrations for day are selected by the skilled person on the basis of the characteristics of the subject to be treated, the severity of the NPC symptoms and on the basis of assessment tests carried out during the treatment.

A further object of the present invention relates to a pharmaceutical composition comprising the NGF or a mutein thereof, as described above, and at least one pharmaceutically acceptable excipient, for use in treating NPC in a subject.

Preferably, said pharmaceutical composition is administered intranasally to said subject.

Preferably, the pharmaceutical composition administration of the invention is a liquid intranasal composition.

Preferably, the pharmaceutical composition according to the present invention comprises an effective amount of the NGF or mutein as described above, and at least one pharmaceutically acceptable excipient suitable for intranasal use, preferably selected from solvents, thickening agents, mucoadhesive agents, buffers, antioxidants, preservatives, and penetration enhancers.

Preferably, the concentration of said NGF or mutein in the liquid intranasal composition according to the invention is between 5 µg/ml and 1 mg/ml, more preferably between 10 µg/ml and 400 µg/ml, even more preferably between 15 µg/ml and 200 µg/ml, most preferably about 20 µg/ml.

According to an equally preferred embodiment, the present invention relates to a brain-derived nerve factor (BDNF), for use in the treatment of Niemann Pick Type C (NPC) disease in a subject, preferably administered intranasally to the subject.

Preferably, said subject is a human subject.

Preferably said BDNF is human BDNF, more preferably it is recombinant human BDNF (rhBDNF).

The evaluation of the administration frequency and dosage are within the knowledge and expertise of the skilled person.

The present invention also relates to a pharmaceutical composition comprising BDNF as defined above, and at least one pharmaceutically acceptable excipient, for use in the treatment of Niemann Pick Type C (NPC) disease in a subject, preferably a human subject.

Preferably, the above composition is administered intranasally to the subject, and it is more preferably a liquid intranasal composition.

Preferably, the at least one pharmaceutically acceptable excipient is selected from solvents, thickening agents, mucoadhesive agents, buffers, antioxidants, preservatives, and penetration enhancers.

Preferably, the concentration of BDNF in the liquid intranasal composition according to the invention is between 5 µg/ml and 1 mg/ml, more preferably between 10 µg/ml and 400 µg/ml, even more preferably between 15 µg/ml and 200 µg/ml.

The pharmaceutical compositions according to the invention may be suitably formulated using appropriate methods known in the art or by the method disclosed in Remington's Pharmaceutical Science (recent edition), Mack Publishing Company, Easton Pa.

In a further aspect, the present invention relates to a method for the treatment of NPC in a subject.

Preferably, said method comprises intranasally administering NGF or a mutein thereof to the subject, as described above, in a therapeutically effective amount.

Preferably, in the method according to the invention, said NGF or mutein is administered as described above.

Preferably, said NGF or mutein used in the method of the invention is in form of a pharmaceutical composition, as above described.

According to another embodiment, said method comprises intranasally administering BDNF to the subject, as described above, in a therapeutically effective amount.

Preferably, in the method according to the invention, said BDNF is administered as described above.

Preferably, said BDNF used in the method of the invention is in form of a pharmaceutical composition, as above described.

The invention will be further described in the following examples, which do not limit the scope of the invention described in the claims.

### EXPERIMENTAL SECTION

### Example 1

### Cell cultures and treatments

Dermal fibroblasts from NPC patients (GM18453, GM03123, GM00110, GM17921, GM18411, provided by Coriell Institute for Medical Research) carrying a frequent pathogenic allele encoding the I1061T variant (Millat et al., 1999) and other relevant mutations listed in Table 1, and from wild-type (WT) sex- and age-matched healthy donors (male donor, GM05659; female donor, GM00726, provided by NIGMS Human Genetic Cell Repository housed by Coriell Institute for Medical Research, Camden, NJ, USA) were cultured in Dulbecco's modified Eagle medium (DMEM) containing high glucose supplemented with 15% fetal bovine serum (FBS), 1% L-glutamine, 1% sodium pyruvate, 1% non-essential amino acids, and 1% penicillin-streptomycin (all Sigma-Aldrich/Merck) at 37°C and 5% CO₂.

Characterization and phenotypic data of the above-mentioned NPC cell lines are summerized in Table 1.

**Table 1: Genotypic and phenotypic characterization of NPC cell lines employed in Example 1.**

| **Cell line** | **Identified mutations** | **Characteristics** |
|---|---|---|
| NPC male donor patient: GM17921 | Heterozygote at the NPC1 gene locus: | NPC1 protein with residual functionality and reduced expression, misfolded and recognized as "non-self". |
| | - allele 1 carries a substitution (T>C) at nucleotide 3182 (c.3182T>C) in exon 21, resulting in a missense mutation at codon **[I1061T (ILE1061THR)];** | Bibliography: |
| | | **ILE1061THR:** |
| | | Millat G et al., Am J Hum Genet. 1999 Nov;65(5):1321-9. |
| | - allele 2 carries a substitution (C>T) at nucleoside 1298 | |
| | (c.1298C>T) in exon 8, resulting in a missense mutation at codon 433 **[P433L (PRO433LEU)];** | Park WD et al., Hum Mutat. 2003 Oct;22(4):313-25 |
| | | **PRO433LEU:** |
| | | No data available |
| NPC male donor patient: | Heterozygote at the NPC1 gene locus: | NPC1 protein with residual functionality and reduced expression, misfolded and recognized as "non-self". |
| | - allele 1 carries a missense mutation C>T at nucleotide 709 (709C>T) in exon 6 of the NPC1 gene, resulting in a substitution of a serine for a proline at codon 237 | Bibliography: |
| | | **[Pro237Ser (P237S)]:** |
| | | No data available |
| | **[Pro237Ser (P237S)];** | |
| GM00110 | - allele 2 carries a 6 bp deletion in exon 14: 2215(or 2217)delTCCTTT(or CTTTTC) which results in the in frame deletion of 2 amino acids (740F,741S) | **2215(or2217)delTCCTTT(orCTTTTC):** |
| | | Yamamoto T, et al., J Med Genet. 2000 Sep;37(9):707-12 |
| NPC male donor patient: | Homozygous at the NPC1 gene locus: alleles 1 & 2 carry a substitution (T>C) at nucleotide 3182 (c.3182T>C) in exon 21, resulting in a missense mutation at codon 1061 [ **I1061T (ILE1061THR)];** | NPC1 protein with residual functionality and reduced expression, misfolded and recognized as "non-self". |
| | | Bibliography: |
| | | **ILE1061THR:** |
| GM18453 | | Millat G et al., Am J Hum Genet. 1999 Nov;65(5):1321-9 |
| | | Park WD et al., Hum Mutat. 2003 Oct;22(4):313-25 |
| NPC female donor patient: | Heterozygote at the NPC1 gene locus: | NPC1 protein with residual functionality and reduced expression, misfolded and recognized as "non-self". |
| | - allele 1 carries a substitution (T>C) at nucleotide 3182 (c.3182T>C) in exon 21, resulting in a missense mutation at codon 1061 **[I1061T (ILE1061THR)];** | Bibliography: |
| | | **ILE1061THR:** |
| GM03123 | | Millat G et al., Am J Hum Genet. 1999 Nov;65(5):1321-9. |
| | -allele 2 carries a pathogenic splice site mutation c.1947+5G>C (rs770321568, g.41940G>C), as well as a missense mutation c.709C>T **[p.Pro237Ser (P237S);** | Park WD et al., Hum Mutat. 2003 Oct;22(4):313-25 |
| | | **c.1947+5G>C (rs770321568, g.41940G>C):** |
| | | No data available |
| | | **[p.Pro237Ser (P237S):** |
| | | Salman A. et al., Am J Med Genet A. 2017 Apr;173(4):1038-1040. |
| | | Blom TS et al., Hum Mol Genet. 2003 Feb 1;12(3):257-72. |
| | | Kaminski WE et al., J Inherit Metab Dis. 2002 Sep;25(5):385-9. |
| NPC female donor patient: | Heterozygote at the NPC1 gene locus: | NPC1 protein with residual functionality and reduced expression, misfolded and recognized as "non-self". |
| | - allele 1 carries a substitution (T>C) at nucleotide 3182 **(c.3182T>C)** in exon 21, resulting in a missense mutation at codon 1061 **[I1061T (ILE1061THR)];** | Bibliography: |
| | | **ILE1061THR:** |
| | | Millat G et al., Am J Hum Genet. 1999 Nov;65(5):1321-9. |
| GM18411 | - allele 2 carries a substitution (G>A) at nucleotide 2498 (c.2498G>A) in exon 16, resulting in a nonsense mutation at codon 833 **[W833X (TRP833TER)];** | Park WD et al., Hum Mutat. 2003 Oct;22(4):313-25 |
| | | **TRP833TER:** |
| | | No data available |

All experiments were performed at 60-70% cell confluency and maximally 20 passages. Specifically, NPC fibroblasts were seeded in 6-well plates in DMEM supplemented with 10% FBS; the day after, NPC fibroblasts received no treatment ("CTRL"), rhNGF (a rhNGF solution consisting of: 100 ng/ml rhNGF, 50 µM sodium phosphate monobasic monohydrate, 100 µM sodium chloride, pH 7.2), rhBDNF (a rhBDNF solution consisting of: 500 ng/ml rhBDNF, 250 µM sodium phosphate, 500 µM NaCl, pH 7.0) or vehicle ("VEH") treatment for 24 hours.

The vehicle solution, used in the experimental tests in which rhNGF was the drug treatment, consisted of: 50 µM sodium phosphate monobasic monohydrate, 100 µM sodium chloride, pH 7.2.

The vehicle solution, used in the experimental tests in which rhBDNF was the drug treatment, consisted of: 250 µM sodium phosphate, 500 µM NaCl, pH 7.0.

The WT GM05659, GM00726 fibroblasts were seeded in 6-well plates in DMEM supplemented with 10% FBS, at 60-70% cell confluency and maximally 20 passages, too.

Statistical significance among the experimental groups was evaluated by using one-way ANOVA followed by Tukey's post-hoc test. A p-value ≤ 0.05 was considered to indicate a statistically significant difference. Data analysis and graphic representation were performed by using GraphPad Prism software package (GraphPad Software, San Diego, CA, USA).

### - rhNGF and rhBDNF reduce cholesterol buildup in NPC fibroblasts.

Firstly, the inventors evaluated the ability of rhNGF and rhBDNF to reduce cholesterol buildup in NPC fibroblasts, by staining fibroblasts with filipin, a known naturally fluorescent polyene antibiotic, that binds to free (i.e. unesterified) cholesterol.

In particular, filipin staining was performed on the above-mentioned cultured fibroblasts of wild-type (WT) cell lines GM05659 and GM00726, and NPC GM18453, GM03123, GM00110, GM17921 and GM18411 cell lines subjected to the above-mentioned treatments.

Filipin staining was performed using Filipin complex (F9765, Merck Life Science, Milan, Italy), and a filipin stock solution (10 mg/ml in PBS) was prepared at the time of use.

Then, the cells were fixed in paraformaldehyde (4% solution) for 10 min, and then washed with PBS. Then, the cells were immediately stained with 1 ml of filipin working solution (0.05 mg/ml in PBS) for 2 h at room temperature in the dark. Next, the cells were washed three times with PBS and the coverslips were mounted with the Fluoroshield mounting medium (F6182, Merck Life Science, Milan, Italy) and immediately observed under a confocal microscope (TCS SP8; Leica Microsystems, Wetzlar) equipped with UV filters (excitation 340-380 nm). Images were acquired at 40x magnification. Filipin quantification was calculated as the mean fluorescence intensity per cell area using ImageJ software (National Institutes of Health, Bethesda, MD, USA) for Windows.

The results of rhNGF treatment are reported in Figure 1.

As expected, filipin staining revealed a significant buildup of free cholesterol in NPC CTRL and VEH-treated fibroblasts, when compared to the free cholesterol concentration detected in WT fibroblast.

Surprisingly, rhNGF treatment (100 ng/ml rhNGF) markedly reduced filipin immunofluorescence in all the tested NPC cell lines, thus indicating a significant reduction of intracellular free cholesterol accumulation in rhNGF-treated NPC cells.

The results of rhBDNF treatment are reported in Figure 2.

The rhBDNF treatment (500 ng/ml rhBDNF) led to a statistically significant reduction of free cholesterol in almost all cell lines, thus showing the efficiency of rhBDNF in reducing the intracellular cholesterol accumulation in NPC cells.

As rhNGF was already effective at 100 ng/ml in reducing intracellular free cholesterol accumulation in NPC cells, the inventors tested only the activity of rhNGF in the next experiments reported below.

### - rhNGF promotes the protein expression of NPC1

The ability of rhNGF to modulate the protein expression of NPC1 in two selected NPC cell lines, namely GM03123 and GM18453, was evaluated.

Notably, these two NPC cell lines were selected because they are the most widely characterized for studies aimed at evaluating the efficacy of putative pharmacological treatments for Niemann-Pick type C disease >Pipalia NH, et al., Histone deacetylase inhibitor treatment dramatically reduces cholesterol accumulation in Niemann-Pick type C1 mutant human fibroblasts. Proc Natl Acad Sci USA. 2011 Apr 5;108(14):5620-5.; Arora S. et al., A high-content RNAi-screening assay to identify modulators of cholesterol accumulation in Niemann-Pick type C cells. Assay Drug Dev Technol. 2010 Jun;8(3):295-320).

The expression and localization of NPC1 protein were assessed by Western blot and immunofluorescence analysis on cultured fibroblasts, according to the protocol previously reported in Martella N, et al., Lavender Essential Oil Modulates Hepatic Cholesterol Metabolism in HepG2 Cells. Curr Issues Mol Biol. 2023 Jan 3;45(1):364-378.

NPC1 immunofluorescence (green) was visualized by using an anti-NPC1 primary antibody (Novus, NB400-14855), and an Alexa Fluor 488 anti-rabbit secondary antibody (Thermo Fisher Scientific, A11008).

Immunofluorescence images were obtained by a confocal microscope (TCS SP8; Leica, Wetzlar, Germany), and processed by ImageJ software.

For Western blot, GAPDH was used as housekeeping protein for loading control.

The results of immunofluorescence, expressed by fluorescence intensity (% to WT), and Western blot analysis concerning NPC GM03123 cell line are reported in Figure 3A and Figure 3B, respectively (WT was GM00726 cell line).

These results demonstrated that NPC1 expression is hardly detectable in NPC GM03123 fibroblasts if compared to WT, probably because NPC1 mutated protein is often recognized as "non-self' and, thus, it is degradated.

Instead, the above-mentioned rhNGF treatment was unexpectedly effective in increasing NPC1 expression in NPC cells.

rhNGF treatment also resulted in an NPC1 immunoreactivity more confined at perinuclear organelles, suggesting an improvement in the correct lysosomal localization of NPC1, in respect of NPC1 immunoreactivity observed in NPC CTRL and VEH-treated fibroblasts (data not shown).

Comparable results were also obtained in NPC GM18453 cells, as demonstrated in the immunofluorescence analysis results, expressed by fluorescence intensity (% to WT), shown in Figure 4A, and Western blot analysis results shown in Figure 4B (WT was GM05659 cell line).

### - rhNGF promotes the correct lysosomal localization of NPC1

To further investigate whether the rhNGF treatment was also able to promote the correct lysosomal localization of NPC1 (Li X et al., 3.3 Å structure of Niemann-Pick C1 protein reveals insights into the function of the C-terminal luminal domain in cholesterol transport. Proc Natl Acad Sci USA. 2017 Aug 22;114(34):9116-9121) in cultured NPC GM03123 and GM18453 cell lines, a co-immunofluorescence analysis was carried out according to the following procedure.

The co-immunofluorescence analysis was performed according to the above-mentioned protocol, by adding primary anti-NPC1 antibody (Novus, NB400-14855) and primary anti-LAMP2 antibody (Santa Cruz Biotechnology, sc-18822), which is known to selectively marking the lysosomal cell compartment, and subsequently Alexa Fluor 488 (Thermo Fisher Scientific, A11008) and Alexa Fluor 555 (Thermo Fisher Scientific, A28180) secondary antibodies, to the cell cultures.

As known, LAMP2 is a membrane glycoprotein localized at lysosomes; therefore, it is a common target to visualize lysosomial compartments within the cells.

NPC1 immunofluorescence (green) localization to lysosomes (LAMP2, red) was calculated by using Pearson's correlation index (also known as Pearson's correlation coefficient) related to NPC1 and LAMP2, and provided by Leica Application Suite X (LAS X) software.

Figures 5A reports the results concerning NPC GM03123 cell line (WT was GM00726 cell line). Surprisingly, rhNGF treatment advantageously increased the correct NPC1 localization to lysosomes in NPC fibroblasts, thus promoting the restoration of the proper regulatory network of intracellular cholesterol homeostasis.

Comparable results were shown in Figure 5B, concerning NPC GM18453 cell line; WT was GM05659 cell line.

### - rhNGF reduces the abnormal nuclear localization of SREBP2.

The putative ability of rhNGF to restore the alterations in the expression of cholesterol regulatory proteins, in particular SREBP2 protein and low-density lipoprotein receptor (LDLR), was evaluated in GM03123 cell line.

An immunofluorescence analysis was carried out according to the above protocol, by using anti-SREBP2 (Santa Cruz Biotechnology, sc-13552), and anti-LDLR (Santa Cruz Biotechnology, sc-11824) antibodies for detecting the nuclear SREBP2 and LDLR, respectively, and the results were expressed by fluorescence intensity (% to WT).

The results regarding the nuclear SREBP2 are reported in Figure 6 (WT was GM00726 cell line), and showed that the rhNGF treatment efficiently counteracted the abnormal nuclear localization of the SREBP2 transcriptionally active fragment, thus drastically reducing the activation of cholesterol synthesis and uptake genes in NPC cells.

The results regarding LDLR are reported in Figure 7 (WT was GM00726 cell line) showing that the rhNGF treatment was also surprisingly efficient to reducing the atypical increase in the expression of this protein. In fact, the fluorescence intensity detected in the NPC cells treated with rhNGF is comparable to that detected in WT cells.

By reducing the abnormal increase of the expression of LDLR in NPC cells, rhNGF treatment promotes the reduction of lipid uptake into cells, as LDLR is one of the main responsible of lipid uptake into the cells.

A reduced lipid uptake advantagueosly results in a reduction of the atypical accumulation of free cholesterol in late endosome of NPC cells.

Moreover, as rhNGF treatment increases the correct NPC1 localization to lysosomes in NPC fibroblasts, as shown above, the resulting increase of free cholesterol in cytoplasm indirectly supports the reduction of the cell need of lipid uptake.

## Claims

1. Nerve growth factor (NGF) or a mutein thereof, for use in the treatment of Niemann-Pick type C disease in a subject.

2. NGF or mutein for use as claimed in claim 1, wherein said NGF or a mutein thereof is administered intranasally to said subject.

3. NGF or mutein for use as claimed in claims 1 or 2, wherein said NGF is human NGF, more preferably it is recombinant human NGF.

4. NGF or mutein for use as claimed in claim 3, wherein said human NGF has the amino acid sequence of SEQ ID NO:1 or SEQ ID NO:2.

5. NGF or mutein for use as claimed in claims 1 to 4, wherein said NGF or mutein is administered daily or every two/three days throughout the period of treatment of between 7 and 90 days, preferably between 15 and 60 days.

6. NGF or mutein for use as claimed in claims 1 to 5, wherein the amount of NGF or mutein per each administration is between 5 µg and 1 mg, more preferably between 10 µg and 400 µg, even more preferably between 15 µg and 200 µg, most preferably about 20 µg.

7. A pharmaceutical composition comprising NGF or a mutein thereof, and at least one pharmaceutically acceptable excipient, for use in the treatment of Niemann-Pick type C disease in a subject.

8. A pharmaceutical composition for use as claimed in claim 7, wherein said composition is administered intranasally to said subject.

9. A pharmaceutical composition for use as claimed in claim 7 or 8, wherein said NGF or a mutein thereof is present in the composition at a concentration between 5 µg/ml and 1 mg/ml, more preferably between 10 µg/ml and 400 µg/ml, even more preferably between 15 µg/ml and 200 µg/ml.

10. A pharmaceutical composition for use as claimed in claims 7 to 9, comprising, preferably consisting of, NGF or a mutein thereof, sodium chloride, phosphate buffer and water.

11. A pharmaceutical composition for use as claimed in claims 7 to 10, wherein said NGF is human NGF, more preferably it is recombinant human NGF.

12. A pharmaceutical composition for use as claimed in claim 7, wherein said human NGF has the amino acid sequence of SEQ ID NO:1 or SEQ ID NO:2.

13. Mutein for use as claimed in claims 1 to 6, and said pharmaceutical composition for use as claimed in claims 7 to 12, wherein said mutein has an amino acid sequence of SEQ ID NO:3, SEQ ID NO:4, SEQ ID NO:5 or SEQ ID NO:6.
